# EUROPEAN PATENT APPLICATION

(11) **EP 0 826 393 A1**
(43) Date of publication of application: **04.03.1998**
(21) Application number: 96113757.7
(22) Date of filing: 28.08.1996
(51) Int. Cl.: A61N 5/10

(54) **Combined angioplasty and intravascular radiotherapy method and apparatus**

(71) Applicant: OMNITRON INTERNATIONAL INCORPORATED, Houston, TX 77054 (US)
(72) Inventor: Bradshaw, Anthony J., Missouri City, Texas 77459 (US); Snyder, Wayne W., Houston, Texas 77062 (US); Thornton, Richard T., League City, Texas 77573 (US)
(74) Representative: Haft, von Puttkamer, Berngruber, Czybulka

(57) **Abstract**

Apparatus and methods are provided for relieving a stenosed region of a blood vessel such as a coronary artery using a single catheter that relieves the angioplasty by conventional means and then delivers an easily controllable inherently uniform dosage of radiation to the walls of a blood vessel for preventing restenosis after angioplasty. The apparatus comprises a catheter having an angioplasty balloon surrounded by a second balloon that is inflatable with a liquid containing a suspended radioactive material such as ¹²⁵I or ³²P. The catheter is advanced through the patient until this treatment balloon is disposed in the stenosed region of the blood vessel. The stenosed region is relieved using the angioplasty balloon after which the outer balloon is filled with the radioactive fluid which expands the outer balloon to engage the walls of the blood vessel thereby providing an inherently uniform dosage of radiation to the blood vessel walls. An outer containment balloon is also provided to prevent loss of radioactive fluid in the event the treatment balloon ruptures. The angioplasty balloon may be filled during the radiation treatment to minimize the volume of radioactive fluid necessary to achieve the desired dosage.

## Description

### Background of the Invention

This invention relates generally to treatment of selected tissue by inter-vivo radiation, specifically to radiation treatment of traumatized regions of the cardiovascular system to prevent restenosis of the traumatized region, more specifically to radiation treatment to prevent restenosis of an artery traumatized by percutaneous transluminal angioplasty (PTA).

PTA treatment of the coronary arteries, percutaneous transluminal coronary angioplasty (PTCA), also known as balloon angioplasty, is the predominant treatment for coronary vessel stenosis. Approximately 300,000 procedures were performed in the United States (U.S.) in 1990 ad a estimated 400,000 in 1992. The U.S. market constitutes roughly half of the total market for this procedure. The increasing popularity of the PTCA procedure is attributable to its relatively high success rate, ad its minimal invasiveness compared with coronary by-pass surgery. Patients treated by PTCA, however, suffer from a high incidence of restenosis, with about 35% of all patients requiring repeat PTCA procedures or by-pass surgery, with attendant high cost ad added patient risk. More recent attempts to prevent restenosis by use of drugs, mechanical devices, and other experimental procedures have had limited success.

Restenosis occurs as a result of injury to the arterial wall during the lumen opening angioplasty procedure. In some patients, the injury initiates a repair response that is characterized by hyperplastic growth of the vascular smooth muscle cells in the region traumatized by the angioplasty. The hyperplasia of smooth muscle cells narrows the lumen that was opened by the angioplasty, thereby necessitating a repeat PTCA or other procedure to alleviate the restenosis.

Preliminary studies indicate that intravascular radiotherapy (IRT) has promise in the prevention or long-term control of restenosis following angioplasty. It is also speculated that IRT may be used to prevent stenosis following cardiovascular graft procedures or other trauma to the vessel wall. Proper control of the radiation dosage, however, is critical to impair or arrest hyperplasia without causing excessive damage to healthy tissue. Overdosing of a section of blood vessel can cause arterial necrosis, inflammation and hemorrhaging. Underdosing will result in no inhibition of smooth muscle cell hyperplasia, or even exacerbation of the hyperplasia and resulting restenosis.

U.S. Patent No. 5,059,166 to Fischell discloses a IRT method that relies on a radioactive stent that is permanently implanted in the blood vessel after completion of the lumen opening procedure. Close control of the radiation dose delivered to the patient by means of a permanently implanted stent is difficult to maintain because the dose is entirely determined by the activity of the stent at the particular time it is implanted. Additionally, the dose delivered to the blood vessel is non-uniform because the tissue that is in contact with the individual strands of the stent receive a higher dosage than the tissue between the individual strands. This non-uniform dose distribution is especially critical if the stent incorporates a low penetration source such as a beta emitter.

U.S. Patent No. 5,302,168 to Hess teaches use of a radioactive source contained in a flexible carrier with remotely manipulated windows. H. Böttcher, et al. of the Johann Wolfgang Goerhe University Medical Center, Frankfurt, Germany report in November 1992 of having treated human superficial femoral arteries with a similar endoluminal radiation source. These methods generally require use of a higher activity source than the radioactive stent to deliver an effective dose. Accordingly, measures must be taken to ensure that the source is maintained reasonably near the center of the lumen to prevent localized overexposure of tissue to the radiation source. Use of these higher activity sources also dictates use of expensive shielding and other equipment for safe handling of the source.

The aforementioned application Serial No. Serial No. 08/352,318, incorporated herein by reference, discloses IRT methods and apparatus for delivering an easily controllable uniform dosage of radiation to the walls of the blood vessel without the need for special measures to center the radiation source in the lumen, the need for expensive shielding to protect medical personnel, or the need for expensive remote afterloaders to handle the higher activity sources. The aforementioned application also discloses methods and apparatus for relieving the stenosed region of the blood vessel ad performing the IRT procedure with a single apparatus.

A further advantage may be obtained, however, if a means is provided for extending the IRT treatment area beyond the angioplasty treatment area to irradiate a region proximal and distal of the angioplasty treatment area as well as the angioplasty treatment area itself. By providing for IRT treatment that covers a wider area than the angioplasty treatment area, proper inhibition of smooth muscle cell hyperplasia is more reliably achieved.

### Summary of the Invention

According to the present invention, a single treatment catheter is used to perform all, or at least the final stage of the angioplasty procedure and to perform the entire IRT procedure. The treatment catheter comprises an flexible elongate member having an angioplasty balloon that is surrounded by an IRT treatment balloon. The catheter is advanced through the cardiovascular system of the patient until the balloons are positioned at a target area comprising the stenosed region of the blood vessel. The stenosis is first relieved using the inner angioplasty balloon, then the target tissue is irradiated by filling the IRT treatment balloon with a radioactive fluid until the outer wall of the balloon gently engages the inner wall of the blood vessel.

In one embodiment of the present invention, the radioactive fluid comprises a suspension of a beta emitting material such as ³²P or a photon emitting material such as ¹²⁵I in a fluid carrier. The radiation emitted by such sources is quickly absorbed by surrounding tissue and will not penetrate substantially beyond the walls of the blood vessel being treated. Accordingly, incidental irradiation of the heart and other organs adjacent to the treatment site is substantially eliminated. Because the radioactive fluid has a substantially uniform suspension of radioactive material, the radiation emitted at the surface of the balloon in contact with the target area of the blood vessel is inherently uniform. Accordingly, uniform irradiation of the blood vessel wall is also inherent.

According to an embodiment of the present invention, the outer IRT treatment balloon is made longer than the inner angioplasty balloon. Accordingly, when filled, the IRT treatment balloon will irradiate a section of the blood vessel that extends on both sides beyond the area treated with the angioplasty balloon. This extended IRT treatment area provides a margin of safety to ensure that, even if the catheter shifts slightly during the treatment, the entire traumatized region of the blood vessel will be treated to prevent smooth muscle cell hyperplasia.

The catheter of the present invention may also be equipped with perfusion ports proximal and distal of the balloon to permit blood flow past the balloon when inflated.

According to another embodiment of the present invention, a third balloon is provided that completely envelopes the IRT treatment balloon. This containment balloon acts as a containment vessel in the event the IRT treatment balloon ruptures when filled with the radioactive fluid. In use, prior to filling the treatment balloon with the radioactive fluid, the containment balloon is filled, preferably with a non-toxic radio-opaque liquid, to verify the integrity of the containment balloon. The radio-opaque fluid filled containment balloon may also be used to verify correct positioning of the catheter within the target area of the blood vessel.

After the angioplasty procedure is performed, the angioplasty balloon may be deflated, or left partially inflated. Leaving the angioplasty balloon partially inflated reduces the amount of radioactive fluid that must be used to fill the treatment balloon by occupying space within the IRT treatment balloon. Because of the self-attenuation of the radioactive fluid itself, most of the radioactivity originates at the surface of the treatment balloon. Accordingly, the surface radiation is not reduced substantially as a result of the center being filled with an inert material. Accordingly, the same radiation level can be achieved as if the IRT treatment balloon were completely filled with radioactive fluid, while using substantially less radioactive fluid.

According to another embodiment of the present invention, a proximal and distal blocking balloon are also provided to contain the radioactive fluid in the target area in the event of a total failure of all containment systems.

### Brief Description of the Drawings

The above and other objects, aspects, features and attendant advantages of the present invention will become apparent from a consideration of the ensuing detailed description of presently preferred embodiments and methods thereof, taken in conjunction with the accompanying drawings, in which:
FIG. 1A-1C are plan and cross sectional views of an apparatus according to the present invention;
FIG. 2A-2C are plan and cross sectional views of an alternate embodiment of an apparatus according to the present invention incorporating a containment balloon; and
FIG. 3A-3C are plan and cross sectional views of an alternate embodiment of an apparatus according to the present invention incorporating proximal and distal blocking balloons;

### Description of Preferred Embodiments and Methods

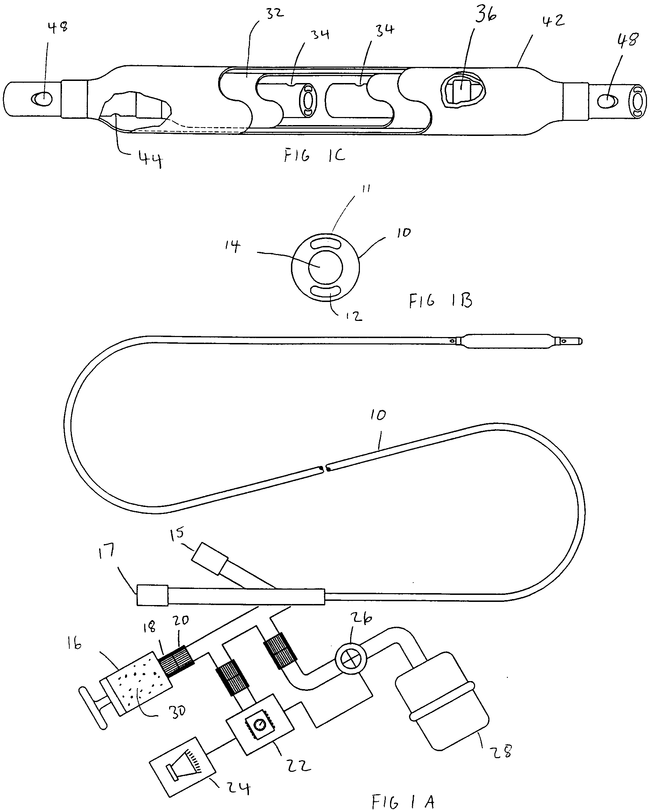

FIGs. 1A and 1B illustrate a suspended-isotope IRT catheter according to the present invention. The IRT catheter comprises shaft 10 having an angioplasty inflation lumen 11, IRT inflation lumen 12, a conventionally formed tip, and may include longitudinal guidewire/injection/perfusion lumen 14. Shielded injector 16, which may be a manual or automated syringe containing a radioactive liquid 30, or a pump connected to a reservoir of radioactive liquid 30, is connected to the proximal end of shaft 10 and is in fluid communication with IRT inflation lumen 12. To prevent possible spillage and corresponding radioactive contamination of the operating room and/or its personnel, the shielded injector 16 is permanently attached to shaft 10, or preferably, injector 16 is equipped with a fail-safe non-detachable connector 18, which cannot be detached from the corresponding receptacle 20 of shaft 10 once it is attached thereto. Non-detachable connector 18 also prevents the radioactive fluid 30 from being discharged from injector 16 until the connector is connected to the receptacle in shaft 10. Connectors having ring-detents and other non-detachable fluid fittings are well known in the art, as are piercing valves and other common methods of preventing fluid flow prior to attachment of a fluid fitting. The proximal end of shaft 10 also includes angioplasty luer fitting 15 in fluid communication with angioplasty inflation lumen 11, and guidewire lumen luer fitting 17 in fluid communication with guidewire lumen 14, through which drugs may be injected directly into the patient's blood stream.

FIG. 1C is an enlarged view of the distal end of the present embodiment of the catheter. Angioplasty balloon 32 comprises a conventional elastic or preferably an inelastic balloon, which may preferably be made from polyethylene terephthalate (PET), polyvinyl chloride (PVC), or other medical grade material suitable for constructing a strong non-compliant balloon. Angioplasty balloon 32 is in fluid communication with angioplasty inflation lumen 11 via ports 34. Immediately inside proximal and distal ends of balloon 32 are markers 36, comprising bands of silver or other suitable x-ray opaque material. Markers 36 aid in the proper positioning of angioplasty balloon 32 within the target area of the blood vessel under fluoroscopy.

IRT Treatment balloon 42 is disposed at the distal end of shaft 10 surrounding angioplasty balloon 32. IRT treatment balloon is an inelastic or preferably an elastic balloon, which is preferably made of latex or other medical grade material suitable for constructing puncture-resistant elastic balloons. IRT treatment balloon 42 is sealed at its proximal and distal ends to catheter shaft 10 in fluid communication with inflation lumen 12 via inflation lumen ports 44.

Immediately adjacent to and outside the ends of IRT treatment balloon 42 are perfusion ports 48, which are in fluid communication with guidewire lumen 14. Perfusion ports are well known in the art as a means of permitting some blood flow past a balloon that is inflated within and otherwise blocking a blood vessel.

In operation, an appropriately sized catheter according to the present invention is selected and positioned within the patient's blood vessel by conventional means so that the balloon is within the target area comprising the stenosed region of the blood vessel. The stenosis is relieved by inflating the angioplasty balloon according to conventional methods. After the angioplasty procedure has been performed, shielded injector 16 is connected to the receptacle at the proximal end of the catheter shaft and the air evacuated from the IRT treatment balloon and the inflation lumen. In the case of a shielded syringe, this is done simply by withdrawing the plunger. The balloon is then filled with the liquid containing the suspended isotope until the outer wall of the balloon gently engages the inner wall of the blood vessel. The balloon is maintained in this inflated state for a predetermined period of time calculated to deliver a effective dose of radiation to the wall of the blood vessel. The fluid is then withdrawn from the balloon and the catheter withdrawn from the patient's body.

To reduce the chances of overpressurizing the treatment balloon and causing a rupture, pressure feedback device 22 is connected to the proximal end of inflation lumen 12. Pressure feedback device 22 may be a pressure gauge, or preferably a solid-state pressure transducer, which in the in the event an overpressure condition is detected, operates an alarm 24 and/or a waste gate 26 that discharges the inflation lumen 12 into a shielded container. Alternately, the solid state pressure transducer may be positioned at the distal end of the inflation lumen to monitor pressure in the balloon directly.

For added safety, prior to filling IRT treatment balloon with radioactive fluid, IRT treatment balloon may be filled with a commonly used non-toxic radio-opaque contrast medium to verify integrity of the IRT treatment balloon. Once the integrity is verified, the contrast medium would be evacuated and shielded syringe 16 connected to the receptacle at the proximal end of the catheter shaft. Although the small amount of contrast medium that would remain in the IRT treatment balloon would dilute the radioactive liquid, the amount of dilution would be measurable and could be compensated.

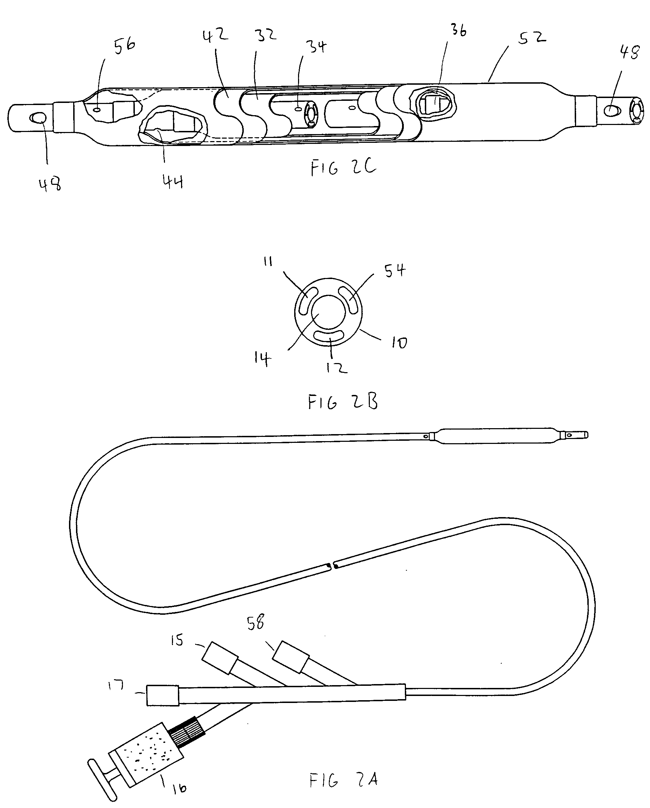

FIGs. 2A-2C illustrate an alternate embodiment of the present invention further including an outer containment balloon 52. Containment balloon 52 is an inelastic or preferably an elastic balloon, which is preferably made of latex or other medical grade material suitable for constructing puncture-resistant elastic balloons. Containment balloon 52 is attached at its proximal and distal ends to shaft 10 and completely surrounds treatment balloon 32. Containment balloon 52 is in communication with containment balloon inflation lumen 54 via containment balloon inflation lumen port 56, which in turn is in fluid communication with containment balloon luer fitting 58 at the proximal end of shaft 10.

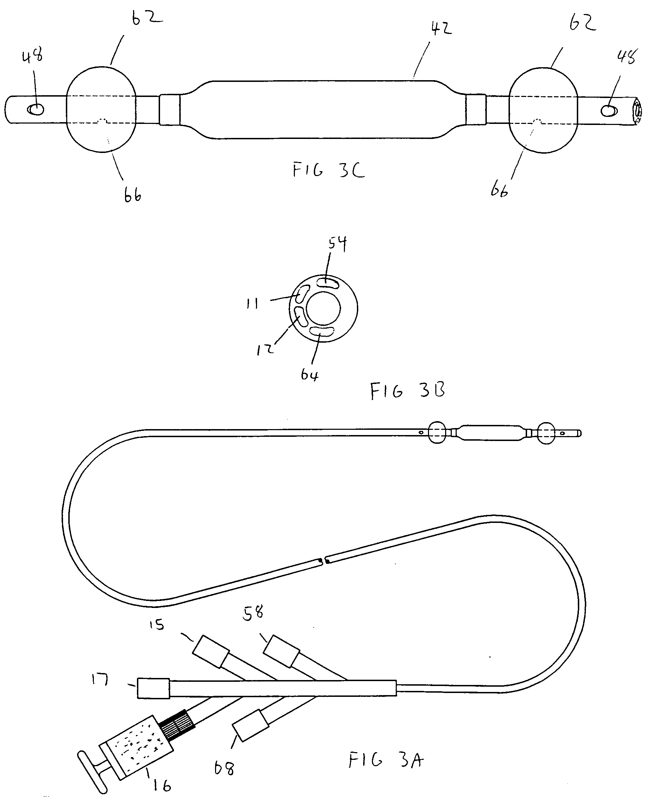

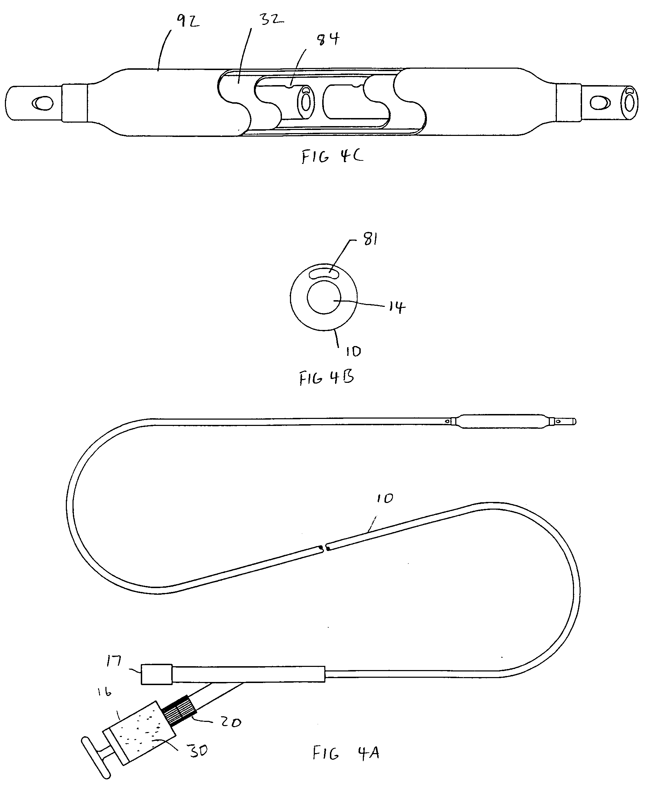

In operation, after the IRT catheter is in position, but before IRT treatment balloon 42 is filled with the radioactive liquid, containment balloon 52 is filled with a commonly used non-toxic radio-opaque contrast medium injected through containment balloon luer fitting 58. The integrity of containment balloon is verified by fluoroscopy, pressure, or other suitable means and, if integrity is confirmed, the radio-opaque liquid is withdrawn and the procedure for injecting the radioactive fluid into treatment balloon 42 carried out. If the integrity of the containment balloon has been compromised (for example by sharp edges in guide catheters, guide wires, stents, etc.) a new catheter is selected and repositioned. By verifying integrity of the containment balloon after the balloon is in position, but before the radioactive fluid is injected, a substantial degree of safety against accidental injection of radioactive fluid into the patient's blood stream is achieved. Where a containment balloon is used (or blocking balloons as discussed with reference to FIGs 3A-3C are used), pressure feedback device 22 may also be used to activate an emergency evacuation system. In the event the pressure feedback device detected a sudden drop in pressure (indicating rupture of the treatment balloon) the pressure feedback device would initiate an immediate withdrawal of all radioactive fluid from the patient, for example by opening a valve to a shielded vacuum accumulator 28.

Several important considerations must be balanced in the design of an apparatus for safely and effectively injecting a radioactive fluid into a patient to irradiate a blood vessel to prevent restenosis. Although ¹²⁵I and ³²P are both emitters of low penetrating radiation suitable for use according to the present invention, ³²P is preferred because it has a half-life of only 14.3 days as compared with the 60 day half-life of ¹²⁵I. A shorter half life renders ³²P safer to use because, in the event of a catastrophic failure involving leakage of radioactive fluid into the patient's blood stream, for a given calculated dose rate, a shorter half life will result in a lesser total body dosage. ³²P is also a relatively pure beta radiation emitter. ³²P has been used in the treatment of chronic leukemia, where it is injected directly into a patient's blood stream. Accordingly, substantial medical knowledge exists as to the effects of ³²P in the blood stream.

In the leukemia treatment, depending on the patient's weight, a suspended radiation source of about 6 to 15 millicuries of ³²P is used. Accordingly, for maximum safety, the preferred suspended-isotope IRT catheter should also use a source of no more than 6 millicuries. Prior experiments have shown that a dose of about 1000 to 3500 rads delivered to the blood vessel wall from a gamma radiation source is effective to inhibit the smooth muscle cell hyperplasia that causes restenosis. For low penetration sources, such as beta radiation emitters, it is believed a dosage up to 5,000 rads may be tolerated. For a 6 millicurie ³²P source to deliver such a dose to the surface of the blood vessel, the balloon must be in position for substantially in excess of one minute, thus necessitating the perfusion ports.

For example, it is estimated that the balloon will absorb approximately 15% of the radiation delivered by the radioactive liquid. Accordingly, to deliver 2000 rads to the blood vessel wall, 2350 rads must be delivered to the inner wall of the balloon. A typical treatment balloon comprises a cylindrical balloon having an internal diameter of 3 millimeters, a length of about 30 millimeters, and an interior volume of approximately 0.2 cubic centimeters. Accordingly, to limit the total source to no more than 6 millicuries, 0.2 cubic centimeters of a liquid having a source concentration of no more than 30 millicuries per cubic centimeter must be used. A 30 millicurie per cubic centimeter source, however, requires about 6 minutes to deliver 2350 rads to the interior of the 3 millimeter diameter treatment balloon and thus requires about 6 minutes to deliver 2000 rads to the interior wall of the blood vessel.

The larger the balloon, the lower the concentration of the radiation source in the liquid must be to maintain the safe limit of 6 millicuries. However, the lower the concentration, the lower the dose rate and the longer the balloon must remain inflated to deliver an effective dose to the blood vessel wall.

To reduce the volume of radioactive fluid that must be used, angioplasty balloon 32 may be left partially or substantially filled during the IRT treatment. Because the fluid near the center of a body of radioactive fluid does not contribute significantly to the radiation emitted from the surface of the body, by leaving the angioplasty balloon partially filled in the center of the IRT treatment balloon, a smaller volume of radioactive liquid can be used without significantly affecting the radiation delivered to the vessel wall. Without the angioplasty balloon acting as an inert filler, to avoid exceeding the 6 millicurie limit, the same size treatment balloon would require a larger volume of lower concentration radioactive fluid, with a commensurately lower dose rate ad longer required treatment interval.

FIGs. 3A-3C illustrate an additional embodiment of the present invention incorporating blocking balloons 62. Blocking balloons 62 are inelastic or preferably elastic balloons, which are preferably made of latex or other medical grade material suitable for constructing puncture-resistant elastic balloons. Blocking balloons 62 are sealed to shaft 10 proximal and distal of treatment balloon 42 between perfusion ports 48, and are in fluid communication with a common blocking balloon inflation lumen 64 via blocking balloon inflation ports 66. Blocking balloon inflation lumen 64 is, in turn, in fluid communication with blocking balloon luer fitting 68 at the proximal end of shaft 10.

In operation, after the angioplasty procedure is completed, blocking balloons 62 are inflated in the blood vessel until the blood flow past the balloons is substantially stopped (the flow of blood in the vessel itself continues through the perfusion ports). The treatment balloon 42 is then inflated with the radioactive fluid for treatment of the blood vessel walls. In the event treatment balloon 42 ruptures and, where present, containment balloon 52 also fails, the radioactive fluid is still contained in the blood vessel between blocking balloons 62. The fluid can then be withdrawn either through any of the inflation lumens that, because of the breach, are in fluid communication with the interior of the blood vessel between the blocking balloons 62, or preferably withdrawn automatically using the emergency evacuation system discussed with reference to FIGs 2A-2C. Blocking balloons may also be used in lieu of containment balloon 52, especially in particularly small lumens where a small profile is desirable.

Thus, the present invention provides safe and effective method and apparatus for combining angioplasty and restenosis prevention by delivering an easily controllable inherently uniform dosage of radiation to the walls of a blood vessel in the region of the blood vessel traumatized by angioplasty, with less chance of ineffective treatment caused by improper positioning of the radioactive source.

Although certain preferred embodiments and methods have been disclosed herein, it will be apparent from the foregoing disclosure to those skilled in the art that variations and modifications of such embodiments and methods may be made without departing from the true spirit and scope of the invention. Accordingly, it is intended that the invention shall be limited only to the extent required by the appended claims and the rules ad principles of applicable law.

## Claims

1. An apparatus for localized intravascular radiotherapy of a blood vessel, such as a coronary artery, comprising:
a catheter, said catheter comprising an elongate member having a proximal and a distal end, said elongate member being sized and of sufficient flexibility to be introducible into a patient's body through a cardiovascular lumen until the distal end is disposed at a target area within the blood vessel, said elongate member further including first and second longitudinal holes therethrough defining an angioplasty balloon inflation lumen and an IRT treatment balloon inflation lumen;
an angioplasty balloon, said angioplasty balloon having an interior chamber, said angioplasty balloon being secured to the distal end of said elongate member with said interior chamber in fluid communication with said angioplasty balloon inflation lumen;
an IRT treatment balloon, said IRT treatment balloon comprising an inflatable balloon having an interior chamber, said IRT treatment balloon being secured to the distal end of said elongate member with said interior chamber surrounding said angioplasty balloon; and
means for introducing a fluid containing a radioactive material into said IRT treatment balloon inflation lumen to fill the interior chamber of said treatment balloon for exposure of said target area to said radioactive material.

2. The apparatus of claim 1 further including means for perfusion of blood past said IRT treatment balloon when said balloon is expanded.

3. The apparatus of claim 1 further including means for detecting pressure in said IRT treatment balloon.

4. The apparatus of claim 1 further including a fluid containing a radioactive material, said fluid being introducible into said balloon by said introducing means.

5. The apparatus of claim 4 wherein said fluid containing a radioactive material comprises a fluid containing a material chosen from the group consisting of iodine and phosphorous.

6. The apparatus of claim 1 wherein said introducing means comprises a shielded syringe.

7. The apparatus of claim 6 wherein said shielded syringe is permanently attached to said catheter.

8. The apparatus of claim 7 wherein said shielded syringe further includes a fail-safe non-detachable fluid coupling and said catheter includes a corresponding fail-safe non-detachable receptacle.

9. The apparatus of claim 1 wherein said introducing means comprises a pump.

10. The apparatus of claim 9 wherein said pump is permanently attached to said catheter.

11. The apparatus of claim 10 wherein said pump further includes a fail-safe non-detachable fluid coupling and said catheter includes a corresponding fail-safe non-detachable receptacle.

12. The apparatus of claim 1 further including means for introducing a contrast medium into said IRT treatment balloon inflation lumen to fill the interior chamber of said IRT treatment balloon for verifying integrity of said IRT treatment balloon.

13. The apparatus of claim 1 further including a containment balloon, said containment balloon comprising a inflatable balloon having an interior chamber, said containment balloon being secured to the distal end of said elongate member with said interior chamber surrounding said IRT treatment balloon.

14. The apparatus of claim 12 wherein said elongate member further includes a longitudinal hole therethrough defining a containment balloon inflation lumen, the interior chamber of said containment balloon being in fluid communication with said containment balloon inflation lumen.

15. The apparatus of claim 13 further including means for introducing a contrast medium into said containment balloon inflation lumen to fill the interior chamber of said containment balloon for verifying integrity of said containment balloon.

16. The apparatus of claim 13 further including means for perfusion of blood past said containment balloon when said containment balloon is expanded.

17. The apparatus of claim 1 further including a first and second blocking balloon each having an interior chamber, said first blocking balloon being secured to the distal end of said elongate member distal of said treatment balloon, said second blocking balloon being secured to the distal end of said elongate member proximal of said treatment balloon; and
wherein said elongate member further includes a blocking balloon inflation lumen, said blocking balloon inflation lumen comprising a longitudinal hole through said elongate member, the interior chambers of said first and second blocking balloons being in fluid communication with said blocking balloon inflation lumen.

18. A method of relieving a stenosis and preventing restenosis of a selected portion of a blood vessel in a patient comprising:
selecting a catheter comprising an elongate member having a distal end, said catheter including an angioplasty balloon and an IRT treatment balloon secured to said distal end, said IRT treatment balloon comprising an inflatable balloon substantially surrounding said angioplasty balloon, said catheter being sized and of sufficient flexibility for introduction into the selected portion of the blood vessel;
advancing said catheter along a cardiovascular lumen of the patient until said IRT treatment balloon is disposed in the selected portion of the blood vessel;
inflating said angioplasty balloon to relieve a stenosed region of said blood vessel;
inflating said IRT treatment balloon with a liquid containing a radioactive material;
maintaining said IRT treatment balloon inflated with said liquid for a predetermined period of time;
withdrawing said liquid from said treatment balloon to allow said treatment balloon to collapse; and
withdrawing said catheter from the patient.

19. The method of claim 18 further including inflating said IRT treatment balloon with a radio-opaque contrast medium to verify integrity of said containment balloon prior to inflating said IRT treatment balloon with said liquid containing a radioactive material.

20. The method of claim 18 wherein said catheter further includes a containment balloon disposed about said treatment balloon and further including:
inflating said containment balloon with a radio-opaque contrast medium to verify integrity of said containment balloon prior to inflating said IRT treatment balloon.
